# EUROPEAN PATENT APPLICATION

(11) **EP 3 981 473 A1**
(43) Date of publication of application: **13.04.2022**
(21) Application number: 20818580.1
(22) Date of filing: 29.05.2020
(51) Int. Cl.: A61P 35/00, A61K 9/14, A61K 47/02, C12N 15/113, A61K 31/7105

(54) **THERAPEUTIC AGENT FOR CANCER**

(30) Priority: 05.06.2019 JP 2019105500
(71) Applicant: Cancerstem Tech Inc., Ibaraki-shi, Osaka 567-0085 (JP); Osaka University, Suita-shi, Osaka 565-0871 (JP)
(72) Inventor: YAMAMOTO, Hirofumi, Suita-shi, Osaka 565-0871 (JP); WU, Xin, Ibaraki-shi, Osaka 567-0085 (JP)
(74) Representative: Hoffmann Eitle
(86) International application number: PCT/JP2020/021318
(87) International publication number: WO 2020/246380

(57) **Abstract**

An object of the present invention is to provide a nucleic acid medicine for cancer treatment that makes it possible to treat cancer effectively. miR-4711-5p can suppress the expression of KLF5, TFDP1, and MDM2, as a result suppressing the proliferation, infiltration, and migration of cancer cells effectively, inducing apoptosis and G1 arrest, and also lowering sternness.

## Description

### TECHNICAL FIELD

The present invention relates to an agent for use in a method of treating cancer. More specifically, the present invention relates to an agent for use in a method of treating cancer that exhibits an excellent antitumor effect and can also reduce the sternness of cancer stem cells.

### BACKGROUND ART

miRNAs are small RNAs consisting of 18 to 24 nucleotides and are widely present in eukaryotes, and the presence of several thousands miRNAs in humans has been revealed. miRNAs are short endogenously expressed RNAs which have been first reported in 1993. RNA having a loop structure called pri-miRNA is transcribed from DNA. The loop is cleaved by an enzyme to create a pre-miRNA. This pre-miRNA is transported out of the nucleus, and an miRNA sequence of about 20 to 25 bases is cut out by a Dicer. This is incorporated into a complex of ribonucleic acid and Argonaute protein called RNA-induced silencing complex (RISC) to form an miRNA-RISC complex, and binds to 3'UTR of mRNA to suppress genetic expression. Since the binding between miRNA and mRNA is incomplete, the number of target genes is not limited to one, and it is an important feature that it is possible to regulate a plurality of genes as targets. In addition, miRNAs play an important role in the regulation of genetic expression in vivo, and it has also been revealed that abnormalities in the regulation system of miRNAs are involved in the causes and progress of many diseases. In particular, various miRNAs that show relevance to the development and progression of cancer have been elucidated, and are attracting attention as bearers of nucleic acid medicines for cancer treatment.

On the other hand, cancer cells have a self-multiplication ability, and have a property that the cancer cells can infiltrate surrounding tissues or metastasize to distant tissues. However, it is known that not all cancer cells forming cancer tissue have these properties, but cancer cells that cause cancer to develop or progress cancer are cancer stem cells that are present very slightly among cancer cells. Similarly to normal stem cells, cancer stem cells exhibit undifferentiated surface properties, have self-renewal ability and multipotency, and have properties of producing all cancer cells constituting cancer tissues in various differentiation stages. That is, it is considered that cancer stem cells are sources that generate a large number of cancer cells by differentiation while maintaining the same cells as themselves by self-replication in cancer tissues. Therefore, it is considered that setting cancer stem cells themselves as treatment targets leads to radical cure of cancer.

In addition, KLF5 is one of KLF families, which are zinc finger transcription factors. It has been revealed that KLF5 is strongly expressed in the crypt in normal intestinal tracts, and KLF5 is widely expressed in cancer sites to enhance Wnt/Catnb signaling, resulting in promoting the growth of cancer cells (Non-Patent Document 1). In addition, it has also been reported that when Klf5 is deleted in Lgr5-positive cells or mice, no tumor is formed (Non-Patent Document 1).

### PRIOR ART DOCUMENT

### NON-PATENT DOCUMENT

Non-Patent Document 1: Takeo Nakaya et al., Cancer Res; 74(10) May 15, 2014

### SUMMARY OF THE INVENTION

### PROBLEMS TO BE SOLVED BY THE INVENTION

As described above, KLF5 is a necessary element for cancerization and has been suggested to be associated with cancer stem cells. However, conventionally, no miRNA capable of binding to KLF5 and effective for treating cancer has been found. In addition, as shown in Test Example 2, there are many miRNAs that can bind to KLF5 but exhibit no growth inhibitory effect on cancer cells. Under such circumstances, an object of the present invention is to provide a nucleic acid medicine for cancer treatment that enables effective treatment of cancer.

### MEANS FOR SOLVING THE PROBLEM

As a result of diligent investigations to solve the above problems, the present inventors have found that miR-4711-5p has a property of not only binding to mRNA of KLF5 involved in cancerization but also binding to mRNA of TFDP1, which is important for transition of the cell cycle from G1 phase to S phase. Furthermore, confirmation has been made that miR-4711-5p suppressed the expression of MDM2 that suppresses p53, which is important for cell apoptosis and cell cycle regulation. It has been found that miR-4711-5p can suppress the expression of KLF5, TFDP1, and MEM2, and as a result, can effectively suppress the growth, invasion, and migration of cancer cells, can keep the cells in the G1 phase, and can further induce the apoptosis of cancer cells. In addition, it has also been found that miR-4711-5p has an action of reducing the sternness of cancer stem cells and has been effective for suppressing the growth of cancer stem cells. The present invention has been completed by further conducting investigations based on these findings.

That is, the present invention provides inventions of the following aspects.
Item 1. An agent for use in a method of treating cancer including miR-4711-5p as an active ingredient.
Item 2. The agent according to Item 1, wherein the miR-4711-5p is a mature miRNA, pri-miRNA, or pre-miRNA.
Item 3. The agent according to Item 1 or 2, wherein the miR-4711-5p is complexed to a carbonate apatite particle.
Item 4. An agent for use in a method of suppressing growth of cancer stem cells including miR-4711-5p as an active ingredient.
Item 5. Use of miR-471 1-5p for the manufacture of an agent for use in a method of treating cancer.
Item 6. miR-4711-5p for use in a method of treating cancer.
Item 7. A method for treating cancer, including administering a therapeutically effective amount of miR-4711-5p to a cancer patient.
Item 8. Use of miR-4711-5p for the manufacture of an agent for use in a method of suppressing growth of cancer stem cells.
Item 9. miR-4711-5p for use in a method of suppressing growth of cancer stem cells.
Item 10. A method for suppressing growth of cancer stem cells, including administering a therapeutically effective amount of miR-4711-5p to a cancer patient to suppress growth of cancer stem cells.

### ADVANTAGES OF THE INVENTION

The cancer therapeutic agent of the present invention binds not only to mRNA of KLF5 involved in cancer stem cells but also to mRNA of TFDP1 necessary for transition of the cell cycle from the G1 phase to the S phase or to mRNA of MDM2 that suppresses p53, the cancer therapeutic agent exhibits an excellent antitumor effect, the cancer therapeutic agent suppresses the growth, invasion, and migration of cancer cells, and the cancer therapeutic agent can effectively induce the apoptosis of cancer cells. In addition, since the cancer therapeutic agent of the present invention can also reduce the sternness of cancer stem cells, the cancer therapeutic agent is capable of suppressing the growth of cancer stem cells, and the clinical usefulness of the cancer therapeutic agent as a nucleic acid medicine for cancer treatment is extremely high.

### BRIEF DESCRIPTION OF DRAWINGS

Fig. 1 is a diagram showing the results of a growth test of colorectal cancer cell lines (DLD-1, HCT116, and HT29) transfected with 41 candidate miRNAs.
Fig. 2 is a diagram showing the results of measuring an expression level of KLF5 mRNA in colorectal cancer cell lines (DLD-1 and HCT116) transfected with miR-4711-5p.
Fig. 3 is a diagram showing the results of measuring the expression level of KLF5 (protein) in colorectal cancer cell lines (DLD-1 and HCT116) transfected with miR-4711-5p.
Fig. 4 is a diagram showing the results of confirming the binding between miR-4711-5p and KLF5 by a dual luciferase assay.
Fig. 5 is a diagram showing the results of a cell growth test of colorectal cancer cell lines (DLD-1 and HCT116) transfected with miR-4711-5p.
Fig. 6 is a diagram showing the results of performing an invasion assay of colorectal cancer cell lines (DLD-1 and HCT116) transfected with miR-4711-5p.
Fig. 7 is a diagram showing the results of evaluating the cell migration ability of colorectal cancer cell lines (DLD-1 and HCT116) transfected with miR-4711-5p.
Fig. 8 is a diagram showing the results of measuring the proportion of cells stained with Annexin V, i.e., cells undergoing apoptosis, in colorectal cancer cell lines (DLD-1 and HCT116) transfected with miR-4711-5p.
Fig. 9 is a diagram showing the results of measuring the expression levels of p53, survivin, cPARP, and cCaspase3 in colorectal cancer cell lines transfected with miR-4711-5p (DLD-1 and HCT116).
Fig. 10 is a diagram showing the results of measuring mRNA amounts of KLF5, CD44v9, LGR5, BMI1, and LRIG1 in colorectal cancer cell lines (DLD-1 and HCT116) transfected with miR-4711-5p.
Fig. 11 is a diagram showing the results of measuring the proportion of CD44v9-positive cells in a colorectal cancer cell line (DLD-1) transfected with miR-4711-5p.
Fig. 12 is a diagram showing the results of measuring reactive oxidant species activity in a colorectal cancer cell line (DLD-1) transfected with miR-4711-5p.
Fig. 13 is a diagram showing the results of evaluating the sphere forming ability in colorectal cancer cell lines (DLD-1 and HCT116) transfected with miR-4711-5p.
Fig. 14 is a diagram showing the results of performing Ingenuity Pathways Analysis using the results of the next generation sequence of colorectal cancer cell lines (DLD-1) transfected with miR-4711-5p or negative control.
Fig. 15 is a diagram showing the results of a cell cycle assay performed on colorectal cancer cell lines (DLD-1 and HCT116) transfected with miR-4711-5p.
Fig. 16 is a diagram showing the results of measuring the expression levels of proteins (TFDP1, E2F1, Rb, CCNE, CDK2, p53, p21, p27, CCND1, CDK2, CDK6, and MDM2) involved in the regulation of the G1 phase/S phase checkpoint and β-actin (ACTB) at protein level in colorectal cancer cell lines (DLD-1 and HCT116) transfected with miR-4711-5p.
Fig. 17 is a diagram showing the results of confirming the binding between miR-4711-5p and TFDP1 by a dual luciferase assay.
Fig. 18 is a diagram showing the results of confirming the binding between miR-4711-5p and MDM2 by a dual luciferase assay.
Fig. 19 is a diagram showing the results of measuring tumor sizes over time in a mouse subcutaneous solid tumor model (colorectal cancer cell line DLD-1).
Fig. 20 is a diagram showing the results of observation of a tumor excised from a mouse on Day 14 in a mouse subcutaneous solid tumor model (colorectal cancer cell line DLD-1).
Fig. 21 is a diagram showing the results of measuring body weights of mice over time in a mouse subcutaneous solid tumor model (colorectal cancer cell line DLD-1).
Fig. 22 is a diagram showing the results of hematoxylin-eosin staining and observation of tissues excised from a mouse on Day 14 in a mouse subcutaneous solid tumor model (colorectal cancer cell line DLD-1).
Fig. 23 is a diagram showing the results of measuring the amount of miR-4711-5p in a tumor in a mouse subcutaneous solid tumor model (colorectal cancer cell line DLD-1).
Fig. 24 is a diagram showing the results of measuring mRNA amounts of KLF5, TFDP1, MDM2, and ACTB in a tumor in a mouse subcutaneous solid tumor model (colorectal cancer cell line DLD-1).
Fig. 25 is a diagram showing the results of measuring the expression levels of KLF5, TFDP1, MDM2, and ACTB at the protein level in a tumor in a mouse subcutaneous solid tumor model (colorectal cancer cell line DLD-1).
Fig. 26 is a diagram showing the results of TUNEL assay of tumor tissues in a mouse subcutaneous solid tumor model (colorectal cancer cell line DLD-1).
Fig. 27 is a diagram showing the results of TUNEL assay of tumor tissues in a mouse subcutaneous solid tumor model (colorectal cancer cell line DLD-1).
Fig. 28 is a diagram showing the results of measuring the protein amounts of cPARP, cCaspase3, and ACTB in a tumor in a mouse subcutaneous solid tumor model (colorectal cancer cell line DLD-1).
Fig. 29 is a diagram showing the results of a cell growth test of colorectal cancer cell lines (DLD-1 and HCT116) transfected with miR-4711-5p, miR-21-5p, miR-152-5p, miR-153-3p, miR-448-3p, and miR-34a. The vertical axis indicates absorbance at 450 nm.
Fig. 30 is a diagram showing the results of transfecting colorectal cancer cells obtained from a colorectal cancer patient with miR-4711-5p and miR-34a and observing the state of the cells after 72 hours.
Fig. 31 is a diagram showing the results of transfecting colorectal cancer cells obtained from a colorectal cancer patient with miR-4711-5p and miR-34a and conducting a cell growth test.

### EMBODIMENTS OF THE INVENTION

### 1. Cancer therapeutic agent

The cancer therapeutic agent of the present invention includes miR-4711-5p as an active ingredient. In the following, the cancer therapeutic agent of the present invention will be described in detail.

### [Active ingredients]

In the cancer therapeutic agent of the present invention, miR-4711-5p is used as an active ingredient. miR-4711-5p is a human-derived miRNA, and its base sequence is 5'-UGCAUCAGGCCAGAAGACAUGAG-3' (SEQ ID NO: 1) when the miR-4711-5p is a mature miRNA.

In the cancer therapeutic agent of the present invention, miR-4711-5p used as an active ingredient may be a mature miRNA, a hairpin-type precursor miRNA (pri-miRNA), or a pre-miRNA in which a part of the pri-miRNA is cleaved. The pri-miRNA and the pre-miRNA undergo intracellular processing to become mature miRNAs. In addition, the miRNAs may form a double-stranded precursor including an RNA having a complementary base sequence. The double-stranded precursor is separated into double strands in the cancer cell to release the mature miRNA. In addition, the base sequence of the hairpin-type precursor miRNA or the pre-miRNA may be set so as to generate a polynucleotide including the base sequence shown in SEQ ID NO: 1 as a mature miRNA, and such a base sequence can be appropriately set by those skilled in the art.

The miRNA used as an active ingredient may be subjected to various modifications generally applied to nucleic acids as necessary in order to impart decomposition resistance to enzymes and the like. Examples of such modification include modification of a sugar chain moiety such as 2'-O methylation, modification of base moieties; and modification of a phosphoric acid moiety such as amination, lower alkyl amination, and acetylation.

### [Application target]

The cancer type to which the cancer therapeutic agent for cancer stem cells of the present invention is applied is not particularly limited, and examples of the cancer types include solid cancers such as colorectal cancer, esophageal cancer, colonic cancer, gastric cancer, rectal cancer, liver cancer, pancreatic cancer, lung cancer, breast cancer, bladder cancer, prostate cancer, cervical cancer, kidney cancer, brain tumor, head and neck cancer, bile duct cancer, gallbladder cancer, and oral cancer; and blood cancers such as leukemia and malignant lymphoma. Among these cancer types, solid cancer is preferable, and colorectal cancer is more preferable.

In addition, since the cancer therapeutic agent of the present invention can also suppress the growth of cancer stem cells, an effective therapeutic effect can be recognized even for cancers for which chemotherapy has not been effective, and thus, as an aspect of an application target of the cancer therapeutic agent for cancer stem cells of the present invention, there is a cancer for which chemotherapy has not been effective.

### [Administration method]

A method for administering the cancer therapeutic agent of the present invention is not particularly limited as long as the miR-4711-5p can be delivered to the cancer tissue or cells in vivo, and examples of the method include intravascular (intraarterial or intravenous) injection, continuous infusion, subcutaneous administration, topical administration, intramuscular administration, and intraperitoneal administration. Among these, arteriovenous administration is preferable.

The dose of the cancer therapeutic agent of the present invention is appropriately determined according to the type of cancer stem cells to be applied, the sex, age, symptom, and the like of the patient, and thus the dose cannot be generally determined. However, for example, it is about 1 to 100 mg/m² (body surface area) per day in terms of the amount of mature miRNA of miR-4711-5p.

### [Formulation]

Since the cancer therapeutic agent of the present invention exhibits a cancer therapeutic effect by delivering the miR-4711-5p into cancer stem cells and expressing its function, the cancer therapeutic agent of the present invention is desirably formulated together with an miRNA transfer agent so that the miR-4711-5p is easily delivered into cancer cells. Such an miRNA transfer agent is not particularly limited, and may be, for example, any of carbonate apatite particles, lipofectamine, oligofectamine, RNAi Fect, and the like. Among these miRNA transfer agents, carbonate apatite particles can be accumulated in cancer cells in vivo to efficiently transfer the miR-4711-5p into cancer cells, and thus a preferred aspect of the cancer therapeutic agent of the present invention includes those in which the miR-4711-5p is present in a mixed state with carbonate apatite particles or in a state of composite particles formed by complexing (including) the miR-4711-5p to carbonate apatite particles.

In the following, the carbonate apatite particles used as a transfer agent for the miR-4711-5p will be described.

### (Carbonate apatite particles)

The carbonate apatite has a structure in which a part of the hydroxyl group of hydroxyapatite (Ca₁₀(PO₄)₆(OH)₂) is substituted with CO₃, and is a compound represented by the general formula Ca₁₀₋ₘXₘ(PO₄)₆(CO₃)₁₋ₙYₙ. Here, X is an element capable of partially substituting Ca in carbonate apatite, and examples the element include Sr, Mn, and a rare earth element. m is a positive number of usually 0 or more and 1 or less, preferably 0 or more and 0.1 or less, more preferably 0 or more and 0.01 or less, and still more preferably 0 or more and 0.001 or less. Y is a group or element capable of partially substituting CO₃ in carbonate apatite, and examples the group or element include OH, F, and Cl. n is a positive number of usually 0 or more and 0.1 or less, preferably 0 or more and 0.01 or less, more preferably 0 or more and 0.001 or less, and still more preferably 0 or more and 0.0001 or less.

The average particle diameter of the carbonate apatite particles used in the present invention is not particularly limited as long as the average particle diameter is a size that can be administered into a living body and transferred into cells. Specifically, the average particle diameter of the carbonate apatite particles used in the present invention is usually more than 30 nm, preferably 30 to 3,000 nm, more preferably 30 to 2,000 nm, and particularly preferably 30 to 1,500 nm.

Note that the average particle diameter of the carbonate apatite is a value measured by dynamic light scattering particle measurement (DLS). When there are huge particles (e.g. the particle size is 5 µm or more.) not suitable for measurement using DLS, the particles are removed from the measurement target range. In addition, in the present specification, the particle size means a particle size of independent particles that can be recognized as separate particles at the time of measurement with a scanning probe microscope. Therefore, when a plurality of particles is aggregated, an aggregate of the particles is determined as one particle.

The carbonate apatite particle can be obtained according to a known method. For example, the carbonate apatite particle can be obtained by allowing calcium ions, phosphate ions, and hydrogen carbonate ions to coexist in an aqueous solution. The ion concentrations in the aqueous solution are not particularly limited as long as carbonate apatite particles are formed, and can be appropriately set with reference to the following.

The calcium ion concentration in the aqueous solution is usually 0.1 to 1,000 mM, preferably 0.5 to 100 mM, further preferably 1 to 10 mM.

The phosphate ion concentration in the aqueous solution is usually 0.1 to 1,000 mM, preferably 0.5 to 100 mM, further preferably 1 to 10 mM.

The hydrogen carbonate ion concentration in the aqueous solution is usually 1.0 to 10,000 mM, preferably 5 to 1,000 mM, further preferably 10 to 100 mM.

The sources of calcium ions, phosphate ions, and hydrogen carbonate ions are not particularly limited as long as these ions can be supplied into the aqueous solution, and examples of the sources include water-soluble salts of these ions. Specifically, CaCl₂ can be used as a calcium ion source, NaH₂PO₄·2H₂O can be used as a phosphate ion source, and NaHCO₃ can be used as a carbonate ion source.

The aqueous solution for preparing the carbonate apatite particles may contain components other than the above-described ion sources and other substances as long as the carbonate apatite particles are formed. For example, Ca or CO₃ in carbonate apatite may be partially substituted by adding fluorine ions, chlorine ions, Sr, Mn, or the like to the composition in an aqueous solution. However, the amounts of fluorine ions, chlorine ions, Sr, and Mn to be added are preferably within a range that does not significantly affect the pH solubility and the particle diameter range of the composite particles to be formed. In addition, as the aqueous solution for preparing the carbonate apatite particles, water may be used as a base, and various media for cell culture, buffers, and the like may be used.

In the preparation of the carbonate apatite particle used in the present invention, the mixing order of the ion sources and other substances into the aqueous solution is not particularly limited, and the aqueous solution may be prepared in any mixing order as long as the intended carbonate apatite particle is obtained. For example, a first solution containing calcium ions and other substances is prepared, and a second solution containing phosphate ions and hydrogen carbonate ions is separately prepared, and the first solution and the second solution are mixed to prepare an aqueous solution.

The carbonate apatite particle can be obtained by adjusting the pH of the aqueous solution containing the ions to a range of 6.0 to 9.0 and leaving (incubating) the aqueous solution for a certain period of time. The pH of the aqueous solution at the time of forming the carbonate apatite particles is, for example, 6.5 to 9.0, preferably 6.7 to 8.8, further preferably 6.7 to 8.6, still further preferably 6.8 to 8.5, particularly preferably 7.0 to 8.5, and most preferably 7.1 to 8.0.

The temperature condition of the aqueous solution at the time of forming the carbonate apatite particles is not particularly limited as long as the carbonate apatite particles are formed. However, the temperature condition is usually 0°C or higher, and for example, 4°C or higher, or 37°C.

Although the incubation time of the aqueous solution for forming the carbonate apatite particles is not particularly limited as long as the carbonate apatite particles are formed, it is usually 1 minute to 24 hours, preferably 5 minutes to 1 hour. The presence or absence of particle formation can be confirmed, for example, by observation under a microscope.

In addition, the method for regulating the average particle diameter of the carbonate apatite particles within the above range is not particularly limited, and examples of the method include a method in which a dispersant is added in the process of producing the carbonate apatite particles or after the production of the carbonate apatite particles. The type of the dispersant is not particularly limited as long as the carbonate apatite particles can be dispersed, and any dispersant generally added to a pharmaceutical product may be used, and an example the dispersant is albumin. The dispersant may be used alone or in combination of two or more kinds of dispersants. Although the concentration of the dispersant in the aqueous solution containing carbonate apatite particles is not particularly limited as long as the effect of suppressing refinement and/or re-aggregation is obtained, examples of the concentration include about 0.1 to 500 mg/ml, preferably 1 to 100 mg/ml, and more preferably 1 to 10 mg/ml; or about 0.001 to 10 wt%. In addition, another example of the method for regulating the particle size is a method in which the carbonate apatite particles formed in the aqueous solution are subjected to ultrasonic vibration processing. Examples of the ultrasonic vibration processing include, specifically, processing of bringing an ultrasonic vibrator such as an ultrasonic crusher into direct contact with a sample and applying an ultrasonic wave, and processing in which a liquid (e.g. water) is put into a water tank using an ultrasonic cleaner provided with an ultrasonic vibrator and the water tank (cleaning tank), a container (e.g. a plastic tube) containing carbonate apatite particles is floated in the water tank, and an ultrasonic wave is applied to an aqueous solution containing carbonate apatite particles through the liquid. With such ultrasonic vibration processing, the particle size of the carbonate apatite particles can be easily and efficiently refined to the above range.

The conditions for the ultrasonic vibration processing are not particularly limited as long as the particle diameter can be regulated within a predetermined range. For example, in the case of using an ultrasonic cleaner including an ultrasonic vibrator and a water tank (cleaning tank), the following conditions are exemplified.

Temperature of the water tank: for example, 5 to 45°C, preferably 10 to 35°C, and more preferably 20 to 30°C.

Radio frequency output: for example, 10 to 500 W, preferably 20 to 400 W, further preferably 30 to 300 W, more preferably 40 to 100 W.

Oscillation frequency: for example, 10 to 60 Hz, preferably 20 to 50 Hz, and more preferably 30 to 40 Hz.

Processing time: for example, 30 seconds to 30 minutes, preferably 1 to 20 minutes, more preferably 3 to 10 minutes.

The type of the container containing the carbonate apatite particles to be used at the time of performing ultrasonic vibration processing is not limited as long as the particles can be refined to a predetermined particle diameter range, and containers can be appropriately selected according to the volume of the aqueous solution and the intended purpose. For example, a plastic tube having a volume of 1 to 1,000 ml can be used.

In addition, the ultrasonic vibration processing is preferably performed in the presence of a dispersant (i.e., a state in which the dispersant is added to an aqueous solution containing carbonate apatite particles). This is because the ultrasonic vibration processing is performed in an environment in which the dispersant and the carbonate apatite particles coexist, carbonate apatite nanoparticles having a finer particle size can be obtained, and the re-aggregation of the particles can also be suppressed.

### (Composite particle of miR-4711-5p and carbonate apatite particle)

In a preferred aspect of the cancer therapeutic agent of the present invention, composite particles having miR-4711-5p and carbonate apatite particles complexed are used. The miR-4711-5p is complexed to the carbonate apatite particle as described above, and thus the miR-4711-5p can be accumulated in cancer cells in vivo by the action of carbonate apatite, and the miR-4711-5p can be efficiently introduced into cancer cells. In addition, since the miR-4711-5p can be released from the carbonate apatite particle in the cell after being introduced into the cell, it is also possible to efficiently exhibit the antitumor effect by the miR-4711-5p.

In the present invention, the composite particle of the miR-4711-5p and the carbonate apatite particle refers to a state in which the miR-4711-5p is adsorbed and supported on the carbonate apatite particle by an ionic bond, a hydrogen bond, or the like. The method for forming the composite particle of the miR-4711-5p and the carbonate apatite particle is not particularly limited, and examples of the method include a method in which the composite particle is formed by allowing the miR-4711-5p and the carbonate apatite particle to coexist in an aqueous solution, a method in which the formation of the carbonate apatite particle and the complexation of the miR-4711-5p to the carbonate apatite particle are simultaneously performed by allowing the miR-4711-5p to coexist with calcium ions, phosphate ions, and hydrogen carbonate ions in an aqueous solution for preparing the carbonate apatite particle.

When the formation of the composite particle of the miR-4711-5p and the carbonate apatite particle and the complexation of the miR-4711-5p and the carbonate apatite particle are simultaneously performed, the miR-4711-5p may be added to the aqueous solution used for preparing the carbonate apatite so as to be, for example, 0.1 to 1,000 nM, preferably 0.5 to 500 nM, and more preferably 1 to 200 nM.

In the composite particle of the miR-4711-5p and the carbonate apatite particle, the ratio of the miR-471 1-5p to the carbonate apatite particle is not particularly limited, and may be appropriately set according to the dose of the miR-4711-5p or the like. For example, when 2 mg of the miR-4711-5p is complexed to the carbonate apatite particle, 5 mg of the miR-4711-5p may be added to 2.5L of the aqueous solution for preparing the carbonate apatite particle to simultaneously form the carbonate apatite particle and complex the miR-4711-5p to the carbonate apatite particle.

In addition, in the present invention, when miR-4711-5p complexed to carbonate apatite particles is used, the complex is used in a state of being dispersed in a solvent suitable for administration to a living body. As described above, although the carbonate apatite particles are obtained by dissolving substances to be various ion sources in a solvent such as water, a medium, or a buffer, the carbonate apatite particle dispersion solution thus obtained is not always suitable for administration to a living body (intravascular administration) from the viewpoint of osmotic pressure, buffering capacity, sterility, and the like. Therefore, in order to replace the solvent in which the carbonate apatite particles are dispersed with a solvent suitable for administration to a living body (e.g. physiological saline solution or the like), it is usually necessary to perform an operation of separating the carbonate apatite particles from the solvent by centrifugation, collecting the particles, and replacing the solvent. However, when such an operation is performed, the carbonate apatite particles are aggregated with each other and the particles become huge, and thus the state is conversely changed to a state not suitable for administration to a living body. Therefore, by adding the aggregated carbonate apatite particles to a solvent suitable for administration to a living body and then performing the ultrasonic vibration processing, the composite particles of the miR-4711-5p and the carbonate apatite particles can be dispersed in a solvent suitable for administration to a living body at an appropriate particle diameter (average particle diameter in the above-described range).

In addition, when the miR-4711-5p complexed to the carbonate apatite particles is used in the cancer therapeutic agent of the present invention, desirably, the administration of the cancer therapeutic agent of the present invention is performed immediately after the composite particles of the miR-4711-5p and the carbonate apatite particles are dispersed in the state of fine particles by ultrasonic vibration processing before the particles are aggregated. For example, administration within 1 minute, preferably within 30 seconds after the ultrasonic vibration processing is preferred. However, as described above, when the aggregation of the carbonate apatite particles is suppressed by adding albumin, after the ultrasonic vibration processing, administration may be performed after several minutes to several tens of minutes.

### 2. Agent for suppressing growth of cancer stem cells

Since miR-4711-5p can also reduce the sternness of cancer stem cells to suppress the growth of cancer stem cells, the present invention further provides an agent for suppressing growth of cancer stem cells containing miR-4711-5p as an active ingredient.

The agent for suppressing growth of cancer stem cells of the present invention is a nucleic acid medicine used for suppressing the growth of cancer stem cells, and the active ingredient to be used, the cancer type as application target, the administration method, the formulation, and the like are the same as those in "1. Cancer therapeutic agent" described above.

### EXAMPLE

In the following, the present invention will be described with reference to Examples. However, the present invention is not to be construed as being limited to the following Examples.

### Test Example 1: Extraction of candidate miRNAs

Two databases, miRBase and Target Scan, were used to extract miRNAs that possibly bound to KLF5. Among the extracted miRNAs, those related to any of Wnt/β-catenin signal, Wnt/Ca⁺ signal, and Notch signal were extracted. As a result, 29 miRNAs were extracted from miRBase, and 31 miRNAs were extracted from Target Scan. Since 16 kinds of miRNAs extracted by miRBase overlapped with 16 kinds of miRNAs extracted by Target Scan, a total of 44 kinds of miRNAs were extracted. Among the 44 kinds of miRNAs, three kinds of miRNAs have already been subjected to functional analysis, and thus 41 kinds of miRNAs were selected as candidates excluding them.

### Test Example 2: Screening of miRNAs

Using the 41 candidate miRNAs extracted above, a growth test of colorectal cancer cell lines was performed. Specifically, first, three colorectal cancer cell lines of DLD-1, HCT116, and HT29 were seeded at 5,000 cells/well in wells of a 96-well plate, followed by overnight culture, and then transfected with a candidate miRNA or a negative control miRNA (NC, 5'-AUCCGCGCGAUAGUACGUA-3', SEQ ID NO: 2) using Lipofectamine 2000 (Invitrogen). After 72 hours from the transfection, cell viability (%) was measured with the GloMax-Multi+ Detection System (Promega) using cell counting kit-8 (DOJINDO LABORATORIES).

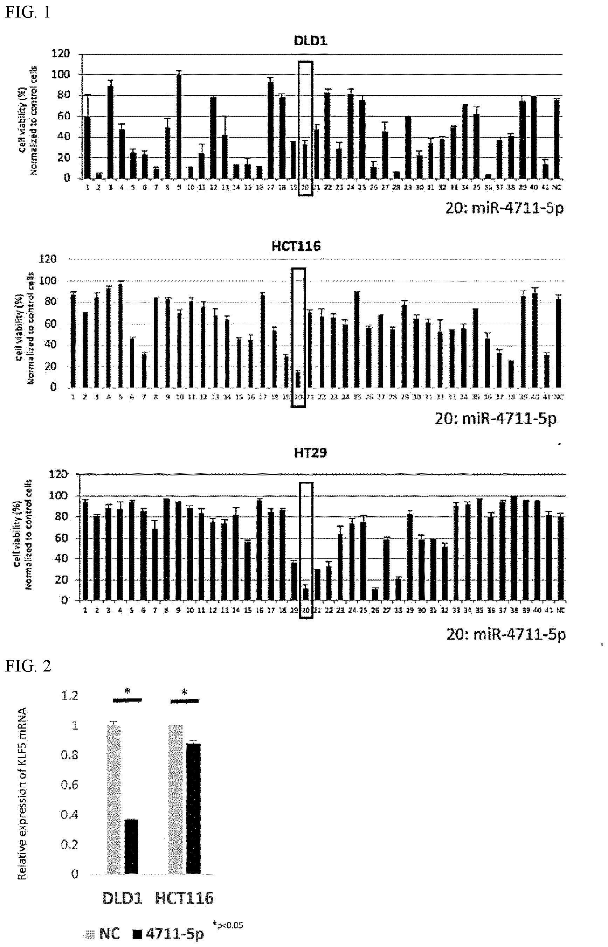

The results are shown in Fig. 1. In Fig. 1, the candidate miRNA of No. 20 is miR-4711-5p. As apparent from Fig. 1, it was confirmed that miR-4711-5p was excellent in the growth inhibitory effect on colorectal cancer cell lines.

### Test Example 3: Analysis of influence of miR-4711-5p on expression level of KLF5 mRNA

The influence on the expression level of KLF5 mRNA was analyzed for miR-4711-5p having an excellent growth inhibitory effect on colorectal cancer cell lines. Specifically, two colorectal cancer cell lines of DLD-1 and HCT116 were seeded in wells of a six-well plate at 1.0 × 10⁵ cells/well, followed by overnight culture, and then transfected with miR-4711-5p or negative control miRNA (NC, SEQ ID NO: 2) using Lipofectamine 2000 (Invitrogen). Cells were collected after 24 hours from the transfection, total RNA was extracted with miRNeasy Kit (Applied Biosystems), and the amount of KLF5 mRNA was measured by PCR.

The results are shown in Fig. 2. As a result, it was confirmed that miR-4711-5p had an action of suppressing the expression of KLF5 at the mRNA level in a colorectal cancer cell line.

### Test Example 4: Analysis of influence of miR-4711-5p on expression level of KLF5 at protein level

The influence of miR-4711-5p on the expression level of KLF5 at the protein level was analyzed. Specifically, two colorectal cancer cell lines of DLD-1 and HCT116 were seeded in wells of a six-well plate at 1.0 × 10⁵ cells/well, followed by overnight culture, and then transfected with miR-4711-5p or negative control miRNA (NC, SEQ ID NO: 2) using Lipofectamine 2000 (Invitrogen). The cells were collected after 48 hours from the transfection, all proteins in the cells were extracted, and the expression level of KLF5 at the protein level was measured by Western blotting.

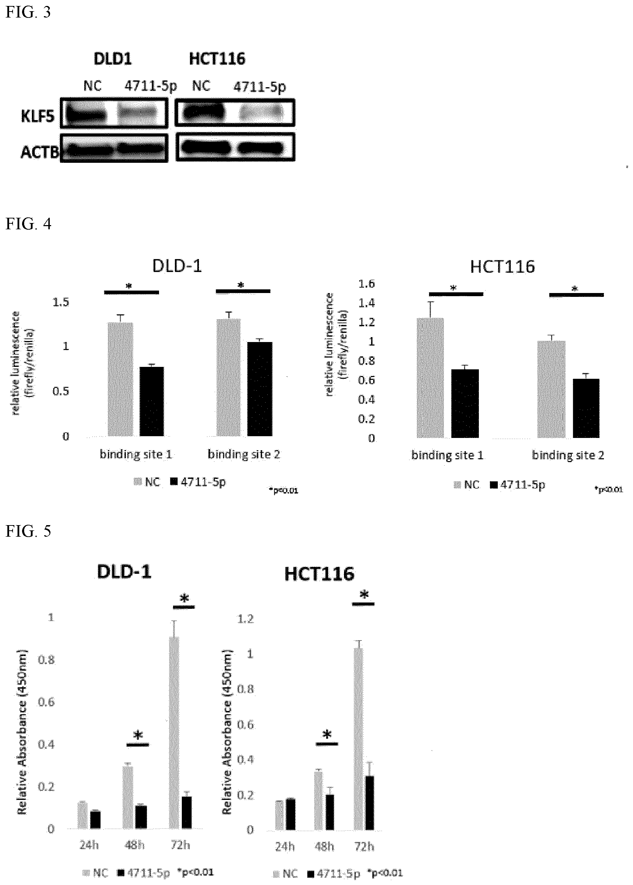

The results are shown in Fig. 3. As a result, it was confirmed that the expression of KLF5 was also suppressed at the protein level in the colorectal cancer cell line transfected with miR-4711-5p.

### Test Example 5: Confirmation of binding between miR-4711-5p and KLF5

In order to confirm whether miR-4711-5p binds to KLF5 in colorectal cancer cell lines transfected with miR-4711-5p, analysis was performed using pmirGLO Dual-Luciferase miRNA Target Expression Vector (Promega). First, using target scan, there were identified binding site 1 (positions 1186 to 1208 of 3'UTR of KLF 5, 5'-AAUAGUAAUGUGAUGCUGAUGCU-3' (SEQ ID NO: 3)) in which strong binding by miR-4711-5p is predicted and binding site 2 (two regions: positions 917 to 939 of 3'UTR of KLF 5, 5'-GACAAUGUUGCAUUUAUGAUGC-3' (SEQ ID NO: 4) and positions 951 to 973 of 3'UTR of KLF 5, 5'-CAAAACGUUGAAUUGAUGAUGCA-5' (SEQ ID NO: 5)) in which weak binding by miR-4711-5p is predicted. A DNA molecule (Insert 1) containing the binding site 1 and a DNA molecule (Insert 2) containing the binding site 2 (two regions) were prepared, and Inserts 1 and 2 were respectively inserted into the multiple cloning sites of pmirGLO Dual-Luciferase miRNA Target Expression Vector.

Two colorectal cancer cell lines of DLD-1 and HCT116 were seeded in wells of a 96-well plate at 1.0 × 10⁴ cells/well, and cultured in a D-MEM medium containing 10% of FBS (fetal bovine serum) for 12 hours. The medium was then replaced with a serum-free Opti-MEM medium, and using Lipofectamine 2000 (Invitrogen), a plasmid into which miR-4711-5p or negative control miRNA (NC, SEQ ID NO: 2) and Insert 1 or 2 were inserted was co-transfected. After 8 hours from the transfection, the medium was replaced with a 10% FBS-containing D-MEM medium, and the cells were further cultured for 24 hours, and then the luciferase protein expression level was measured according to the product protocol.

The results are shown in Fig. 4. As a result, it was confirmed that the expression of luciferase was suppressed by miR-4711-5p, and miR-4711-5p bound to both binding sites 1 and 2.

### Test Example 6: Cell growth test

A cell growth test of a colorectal cancer cell line was performed using miR-4711-5p. Specifically, first, two colorectal cancer cell lines of DLD-1 and HCT116 were seeded in wells of a 96-well plate at 3.0 × 10³ cells/well, followed by overnight culture, and then miR-4711-5p or a negative control miRNA (NC, SEQ ID NO: 2) was transfected using Lipofectamine 2000 (Invitrogen). After 24, 48, and 72 hours from the transfection, the cell survival number was measured with GloMax-Multi+ Detection System (Promega) using cell counting kit-8 (DOJINDO LABORATORIES).

The results are shown in Fig. 5. As a result, it was confirmed that when transfection of miR-4711-5p was performed, it made it possible to significantly suppress the cell growth of DLD-1 and HCT116 after 48 and 72 hours as compared with the negative control.

### Test Example 7: Invasion assay

Invasion assays of colorectal cancer cell lines were performed using miR-4711-5p. Specifically, first, two colorectal cancer cell lines of DLD-1 and HCT116 were seeded in wells of a 6-well plate at 1.0 × 10⁵ cells/well, followed by overnight culture, and then miR-4711-5p or a negative control miRNA (NC, SEQ ID NO: 2) was transfected using Lipofectamine 2000 (Invitrogen). After 24 hours from the transfection, cells were seeded on BD BioCoat Matrigel Invasion Chambers (BD biosciences) and cultured for 48 hours. Subsequently, nuclei were stained with henatoxylin, and the number of infiltrated cells was measured.

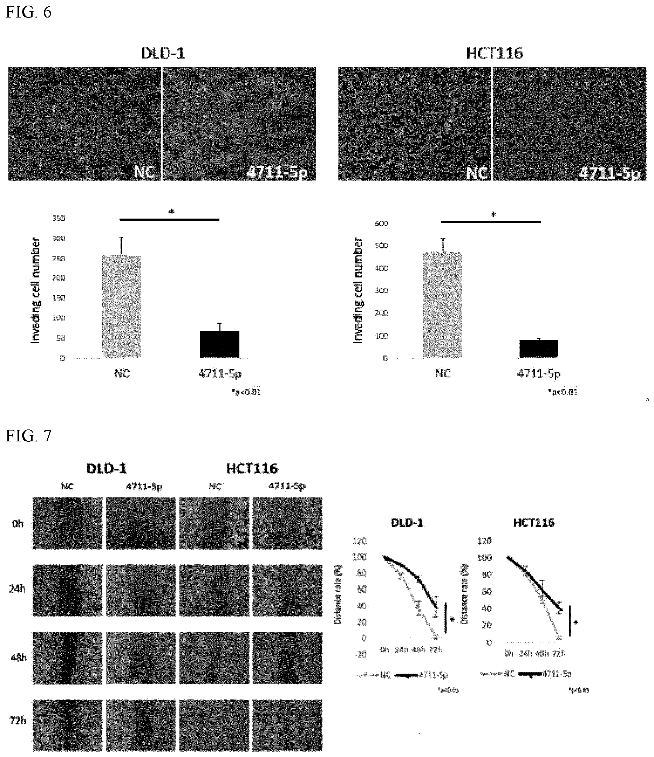

The results are shown in Fig. 6. As a result, it was confirmed that when transfection of miR-4711-5p was performed, the number of infiltrating cells was significantly reduced as compared with the negative control.

### Test Example 8: Evaluation of migration ability

Using miR-4711-5p, the migration ability of colorectal cancer cell lines was evaluated by a wound healing assay. Specifically, first, an insert (ibidi Culture Insert 2 Well (ibidi)) was set in a 24 well plate, and two colorectal cancer cell lines, DLD-1 and HCT116, were seeded in wells at 3.5 × 10⁵ to 4.0 × 10⁵ cells/well, and cultured for 24 hours. Subsequently, the insert was removed to produce a wound, followed by culture, and the wound area was measured after 24, 48, and 72 hours from the removal of the insert.

The results are shown in Fig. 7. As a result, it was confirmed that when transfection of miR-471 1-5p was performed, the wound area was significantly wider than that of the negative control, and the migration ability of cells was reduced.

### Test Example 9: Apoptosis assay

Apoptosis assays of colorectal cancer cell lines were performed using miR-4711-5p. Specifically, first, two colorectal cancer cell lines of DLD-1 and HCT116 were seeded in wells of a 6-well plate at 1.0 × 10⁵ cells/well, followed by overnight culture, and then miR-4711-5p or a negative control miRNA (NC, SEQ ID NO: 2) was transfected using Lipofectamine 2000 (Invitrogen). After 48 hours from the transfection, cells were treated with Alexa Fluor 488 Annexin V/Dead Cell Apoptosis Kit (Thermo Fisher Scientific), and the proportion stained with Annexin V was determined using SH800ZCell Sorter (Sony Biotechnology Inc.). In addition, the cells were collected after 48 hours from the transfection, all proteins in the cells were extracted, and the expression levels of p53, survivin, cPARP (cleaved PARP), cCaspase3 (cleaved Caspase3), and ACTB (β-actin) were measured by Western blotting.

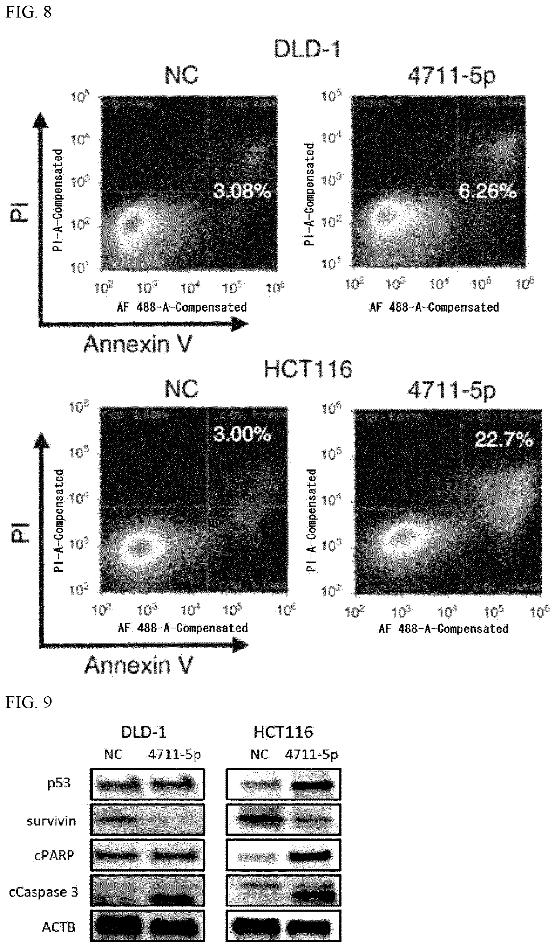

The results of measuring the proportion of cells stained with Annexin V (i.e., cells undergoing apoptosis) are shown in Fig. 8, and the results of measuring the expression levels of p53, survivin, cPARP, cCaspase3, and ACBT are shown in Fig. 9. As a result, it was found that in the colorectal cancer cell line transfected with miR-4711-5p, the number of cells stained with Annexin V increased, and apoptosis was strongly induced. In addition, in HCT116 having wild-type p53, by transfecting with miR-4711-5p, the expression of p53 was enhanced, the expression level of survivin which is an anti-apoptotic protein was decreased, and the expression levels of cPARP and cCaspase3 which are apoptotic proteins were increased. In addition, in DLD-1 having mutant type p53, transfection with miR-4711-5p decreased the expression of survivin and increased the expression level of cCaspase3. That is, from these results, it was revealed that the apoptosis of colorectal cancer cell lines was induced by transfection of miR-4711-5p.

### Test Example 10: Analysis of expression level of stem cell marker

The influence of miR-4711-5p on the expression level of stem cell markers of colorectal cancer cell lines was analyzed. Specifically, first, two colorectal cancer cell lines of DLD-1 and HCT116 were seeded in wells of a 6-well plate at 1.0 × 10⁵ cells/well, followed by overnight culture, and then miR-4711-5p or a negative control miRNA (NC, SEQ ID NO: 2) was transfected using Lipofectamine 2000 (Invitrogen). Cells were collected after 24 hours from the transfection, total RNA was extracted with miRNeasy Kit (Applied Biosystems), and mRNA amounts of KLF5, CD44v9, LGR5, BMI1 and LRIG1 were measured by PCR. In addition, for DLD-1 after 48 hours from the transfection, the amount of CD44v9 on the cell surface was measured by FACS.

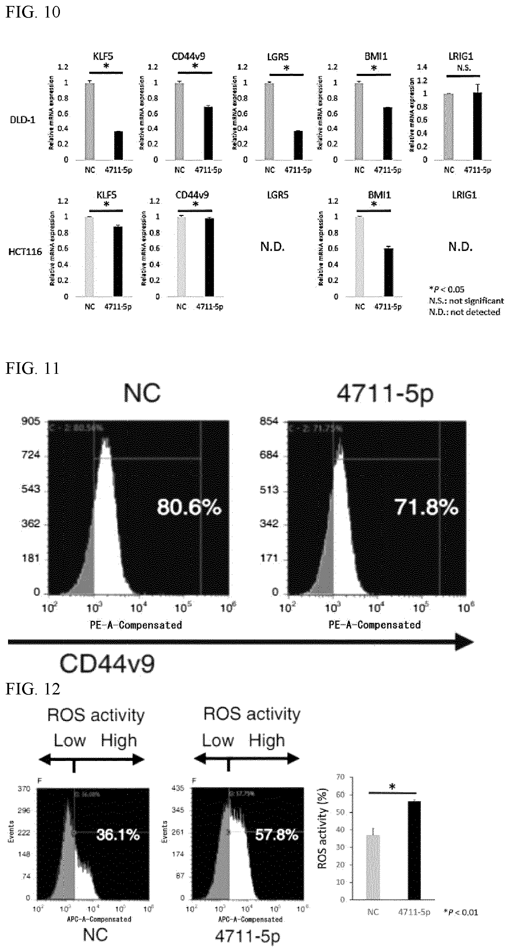

The results of measuring the mRNA amounts of KLF5, CD44v9, LGR5, BMI1, and LRIG1 are shown in Fig. 10, and the results of measuring the amount of CD44v9 on the surface of the DLD-1 by FACS are shown in Fig. 11. As shown from Fig. 10, by transfection of miR-4711-5p, the expression of stem cell markers CD44v9, LGR5, and BMI1 was suppressed in DLD-1, and the expression of stem cell markers CD44v9 and BMI1 was suppressed in HCT116. Also, as shown from Figure 11, transfection of miR-4711-5p reduced CD44v9 present on the cell surface of DLD-1. From the above results, it was revealed that in the colorectal cancer cell line transfected with the miR-4711-5p, the expression of the stem cell marker was suppressed, and the miR-471 1-5p had an action of reducing the sternness of cancer stem cells.

### Test Example 11: Measurement of reactive oxidant species activity

Cells with high sternness have the property of maintaining low reactive oxidant species (ROS) activity. Therefore, the influence of miR-4711-5p on ROS activity of colorectal cancer cell lines was analyzed. Specifically, first, DLD-1 was seeded in a six-well plate at 1.0 × 10⁵ cells/well, followed by overnight culture, and then miR-4711-5p or a negative control miRNA (NC, SEQ ID NO: 2) was transfected using Lipofectamine 2000 (Invitrogen). Cells were collected after 24 hours from the transfection and stained with CellROX Deep Red (thermos fisher scientific) to measure ROS activity by FACS.

The results are shown in Fig. 12. Note that the ROS activity shown in the right diagram of Fig. 12 is the proportion of cells in which the ROS activity is determined to be high. The cut-off values of High and Low were determined using cells that were exposed to no Cell ROX reagent. As a result, it was confirmed that in the colorectal cancer cell line transfected with the miR-4711-5p, the proportion of cells having high ROS activity increased and the miR-4711-5p decreased sternness.

### Test Example 12: Analysis of sphere forming ability

The influence of miR-4711-5p on the sphere forming ability of colorectal cancer cell lines was analyzed. Specifically, first, two colorectal cancer cell lines of DLD-1 and HCT116 were seeded in wells of a 6-well plate at 1.0 × 10⁵ cells/well, followed by overnight culture, and then miR-4711-5p or a negative control miRNA (NC, SEQ ID NO: 2) was transfected using Lipofectamine 2000 (Invitrogen). After 24 hours from the transfection, the cells were collected, and seeded at 1 × 10³ cells/well in 96 well ultra-low attachment plates (Corning Inc., USA.) in a state in which the cells were not aggregated with each other, and cultured for one week. Note that for the culture, a serum-free DMEM/F-12 medium containing EGF of 20 ng/ml, bFGF of 10 ng/ml, and penicillin G of 100 µg/ml was used. After one week, the number of spheroids of 40 µm or more was measured (n = 10).

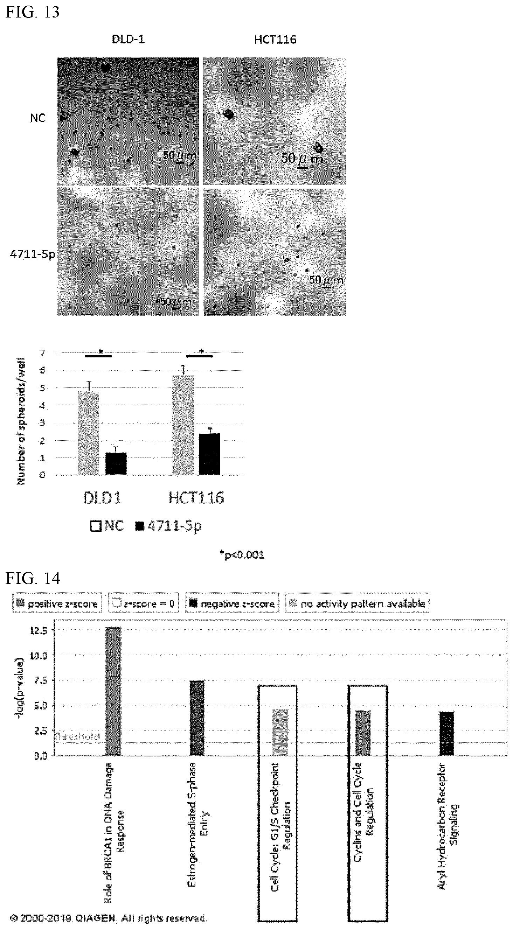

The results are shown in Fig. 13. As a result, it was confirmed that the number of spheroids in a colorectal cancer cell line transfected with miR-471 1-5p was reduced, and it was revealed that miR-4711-5p has an effect of reducing the sphere forming ability of colorectal cancer cell lines.

### Test Example 13: IPA analysis

For colorectal cancer cell lines transfected with miR-4711-5p, Ingenuity Pathways Analysis (IPA) was performed using the result of next generation sequencing. Specifically, first, DLD-1 was seeded in wells of a 6-well plate at 1.0 × 10⁵ cells/well, followed by overnight culture, and then miR-4711-5p or a negative control miRNA (NC, SEQ ID NO: 2) was transfected using Lipofectamine 2000 (Invitrogen). Cells were collected after 36 hours from the transfection and total RNA was extracted with miRNeasy Kit (Applied Biosystems). Subsequently, library preparation was performed using the obtained total RNA, and then next generation sequence analysis of the library was performed. Subsequently, Ingenuity Pathways Analysis was performed using the results of the next-generation sequencing.

The results are shown in Fig. 14. As a result, it was suggested that transition from G1 phase to S phase in the cell cycle was suppressed in the colorectal cancer cell line transfected with miR-4711-5p.

### Test Example 14: Cell cycle assay

Since the result of Test Example 13 suggested that miR-4711-5p suppresses the transition from G1 phase to S phase in the cell cycle, the influence of miR-4711-5p on the cell cycle of a colorectal cancer cell line was analyzed in detail. Specifically, first, DLD-1 was seeded at 1.0 × 10⁵ cells/well in wells of a 6-well plate, followed by overnight culture using a 10% FBS-containing D-MEM medium. Subsequently, the medium was replaced with a FBS-free D-MEM medium, and the cells were cultured for 24 hours. Subsequently, using Lipofectamine 2000 (Invitrogen), the cells were transfected with miR-4711-5p or a negative control miRNA (NC, SEQ ID NO: 2). After 24 hours from the transfection, the medium was replaced with a 10% FBS-containing D-MEM medium. Subsequently, the cells were cultured for 8 hours, and then collected and subjected to a cell cycle assay. Techniques for cell collection and cell cycle assays are as follows.

Cells in the wells were washed with PBS and treated with Trypsin-EDTA to liberate the cells. Subsequently, the cells were suspended in a 10% FBS-containing D-MEM medium and collected, and the cells were placed in a 15 ml tube, followed by centrifugation and supernatant removal. After that, PBS was added and centrifugation was performed again. Subsequently, the supernatant was removed, and 200 µl of a cold 70% aqueous ethanol solution was added dropwise little by little while stirring. After that, stirring was stopped, and 300 µl of a cold 70% ethanol aqueous solution was added while moving through the wall surface of the container, and ice-cooled for 30 minutes. Subsequently, centrifugation and supernatant removal were performed, followed by washing with PBS and centrifugation again. After centrifugation, the supernatant was removed, 500 µl of 0.1 mg/ml RNase was added, and the mixture was incubated at 37°C for 20 minutes. After incubation, centrifugation was performed to remove the supernatant, and then 200 µl of 25 µg/ml propidium iodide was added and incubated for 20 minutes. Subsequently, 300 µl of the FACS flow sheath liquid was added, and analysis was performed by FACS through a cell strainer.

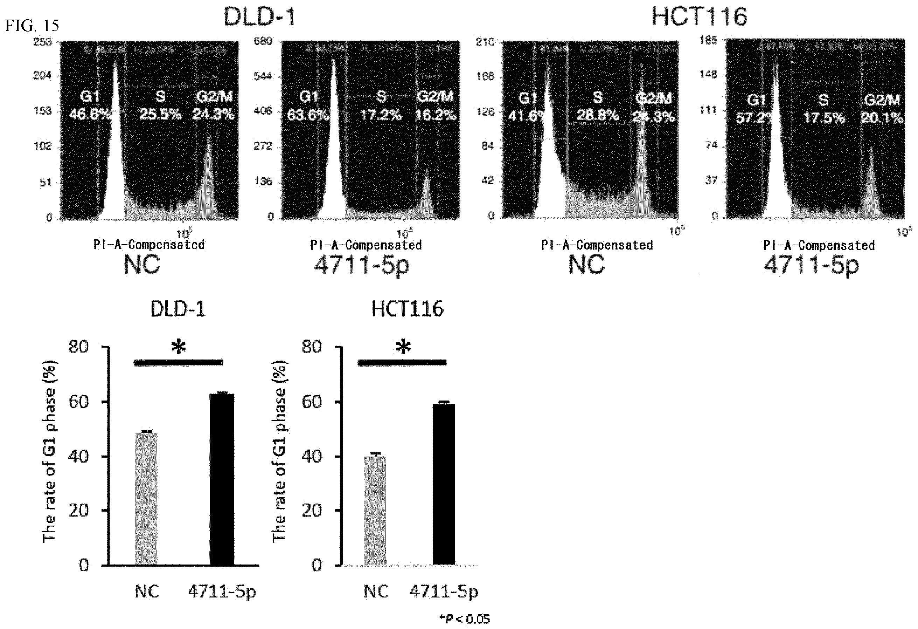

The results are shown in Fig. 15. As a result, it was confirmed that in the colorectal cancer cell line transfected with miR-4711-5p, the proportion of cells in G1 phase was increased, and the transition from G1 to S phase was suppressed.

### Test Example 15: Measurement of expression level of protein involved in regulation of G1 phase/S phase checkpoint

For miR-4711-5p, the influence on the expression level of proteins involved in the regulation of the G1 phase/S phase checkpoint was analyzed. Specifically, two colorectal cancer cell lines of DLD-1 and HCT116 were seeded in wells of a six-well plate at 1.0 × 10⁵ cells/well, followed by overnight culture, and then transfected with miR-4711-5p or negative control microRNA (NC, SEQ ID NO: 2) using Lipofectamine 2000 (Invitrogen). After 48 hours from the transfection, the cells were collected, all proteins in the cells were extracted, and the expression levels of proteins involved in the regulation of the G1 phase/S phase checkpoint (TFDP1, E2F1, Rb, CCNE, CDK2, p21, p27, CCND1, CDK2, CDK6, and MDM2) and β-actin (ACTB) at the protein level were measured by Western blotting.

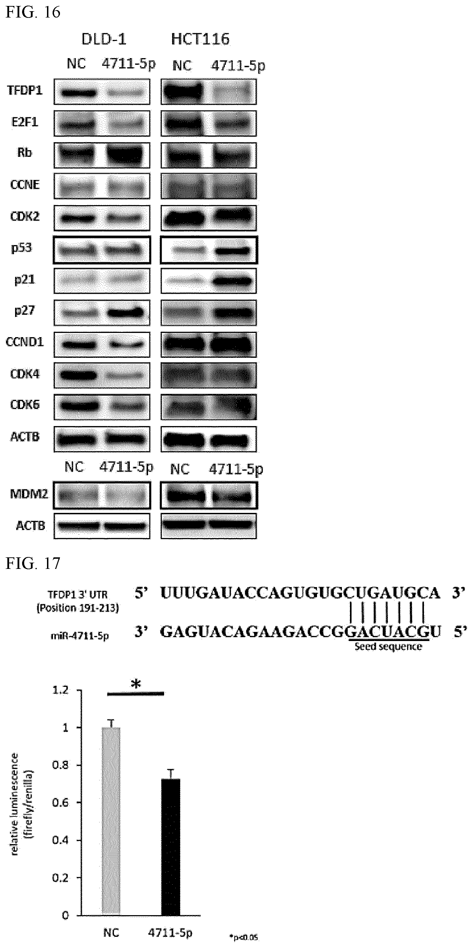

The results are shown in Fig. 16. As a result, in the colorectal cancer cell line transfected with miR-4711-5p, the expression of p21 and p27, which stop the cell cycle, was enhanced, and the expression of TFDP1 and MDM2 was also reduced. In HCT 116 of the p53 wild-type strain, the expression of p53 was enhanced, and the expression of p21 on the downstream was enhanced.

### Test Example 16: Confirmation of binding between miR-4711-5p and TFDP1

In Target Scan, it was suggested that TFDP1 has a binding region of miR-4711-5p. Therefore, in order to confirm whether miR-4711-5p binds to TFDP1 in a colorectal cancer cell line transfected with miR-4711-5p, analysis was performed using pmirGLO Dual-Luciferase miRNA Target Expression Vector (Promega). First, using target scan, a binding site (position 191-213 of 3'UTR of TFDP1, 5'-UUUGAUACCAGUGUGCUGAUGCA-3' (SEQ ID NO: 6)) predicted to be bound by miR-4711-5p was identified. A DNA molecule (insert) containing the binding site was prepared, and the insert was inserted into a multi-cloning site of pmirGLO Dual-Luciferase miRNA Target Expression Vector.

The wells of a 96-well plate was seeded with DLD-1 at 3.0 × 10³ cells/well, and cultured in a D-MEM medium containing 10% of FBS (fetal bovine serum) for 12 hours. Subsequently, the medium was replaced with a serum-free Opti-MEM medium, and using Lipofectamine 2000 (Invitrogen), a plasmid in which miR-4711-5p or a negative control miRNA (NC, SEQ ID NO: 2) and an insert were inserted was co-transfected. After 8 hours from the transfection, the medium was replaced with a 10% FBS-containing D-MEM medium, and after further culturing for 24 hours, luciferase activity was measured according to the product protocol.

The results are shown in Fig. 17. As a result, it was confirmed that the expression of luciferase was suppressed by miR-4711-5p, and miR-4711-5p bound to positions 191 to 213 of 3'UTR of TFDP1.

### Test Example 17: Confirmation of binding between miR-4711-5p and MDM2

In Target Scan, it was suggested that MDM2 has a binding region of miR-4711-5p. Therefore, in order to confirm whether miR-4711-5p binds to MDM2 in a colorectal cancer cell line transfected with miR-4711-5p, an analysis was performed using pmirGLO Dual-Luciferase miRNA Target Expression Vector (Promega). First, using target scan, a binding site (positions 2315 to 2337 of 3'UTR of MDM2, 5'-UAUGGAAUAAAACUACUGAUGCA-3' (SEQ ID NO: 7)) predicted to be bound by miR-4711-5p was identified. A DNA molecule (insert) containing the binding site was prepared, and the insert was inserted into a multi-cloning site of pmirGLO Dual-Luciferase miRNA Target Expression Vector.

The wells of a 96-well plate was seeded with DLD-1 at 3.0 × 10³ cells/well, and cultured in a D-MEM medium containing 10% of FBS (fetal bovine serum) for 12 hours. Subsequently, the medium was replaced with a serum-free Opti-MEM medium, and using Lipofectamine 2000 (Invitrogen), a plasmid in which miR-4711-5p or a negative control miRNA (NC, SEQ ID NO: 2) and an insert were inserted was co-transfected. After 8 hours from the transfection, the medium was replaced with a 10% FBS-containing D-MEM medium, and after further culturing for 24 hours, luciferase activity was measured according to the product protocol.

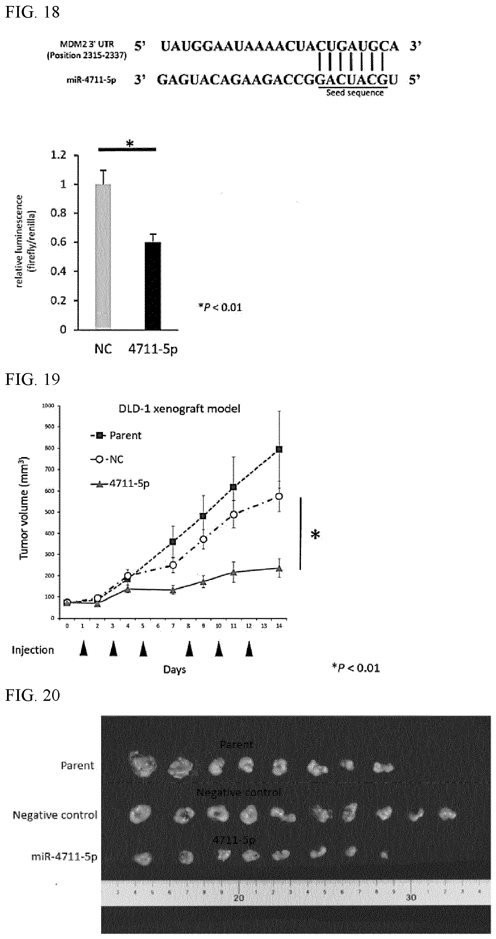

The results are shown in Fig. 18. As a result, it was confirmed that the expression of luciferase was suppressed by miR-4711-5p, and miR-4711-5p bound to positions 2315 to 2337 of the 3'UTR of MDM2.

### Test Example 18: Evaluation of antitumor effect and safety of miR-4711-5p in mouse subcutaneous solid tumor model

### 1. Production of composite particles of miRNA and carbonate apatite

To 100 ml of distilled water, 0.37 g of NaHCO₃, 90 µl of NaH₂PO₄·2H₂O (1 M), and 180 µl of CaCl₂ (1 M) were added in this order for dissolution, and the pH was adjusted to 7.5 with HCl (1 N). This was filtered through a filter having a diameter of 0.2 µm. Per 1 ml of the obtained buffer, 2 µg of miRNA and 4 µl of CaCl₂ (1 M) were mixed and the mixture was incubated in a water bath at 37°C for 30 minutes. After that, the mixture was centrifuged at 15,000 rpm × 5 minutes, and the obtained pellet was dispersed in physiological saline solution (containing 0.5 wt% albumin) to obtain a dispersion of composite particles in which miRNA was encapsulated in carbonate apatite particles. Immediately before the test, the obtained dispersion was subjected to ultrasonic vibration processing for 10 minutes. Note that the ultrasonic vibration processing was performed using a water bath having an ultrasonic vibration function for 10 minutes under the conditions of a radio frequency output of 55 W and an oscillation frequency of 38 kHz by floating the dispersion contained in a plastic container in water set at 20°C. The formulations thus obtained were immediately used for the tests described below.

Note that it is confirmed that composite particles composed of carbonate apatite nanoparticles containing miRNA are produced in the dispersion subjected to ultrasonic vibration processing, and the composite particles contain about 20 µg of miRNA per 1 mg of carbonate apatite.

### 2. Mouse subcutaneous solid tumor model test

On the left and right back of a six to eight week-old BALB/cA nude mouse (Female, manufactured by CLEA Japan, Ltd.), DLD-1 was subcutaneously injected at 5 × 10⁵ cells/tumor to prepare a model mouse having a solid tumor. When the tumor reached a size of about 20 mm³, the mice were randomly divided into three groups of five animals in a miR-4711-5p administration group (miR-4711-5p), five animals in a negative control group (Negative control), and four animals in an untreated group (Parent). In the miR-4711-4711-5p administration group, the time point at which the tumor reached a size of about 20 mm³ was defined as Day 0, and on Day 0, Day 2, Day 4, Day 7, Day 9, and Day 11, carbonate apatite nanoparticles containing iR-4711-5p were intravenously injected from the tail vein so as to be 40 µg/time in terms of miR-5p amount. In the negative control group, carbonate apatite nanoparticles containing a control miRNA (SEQ ID NO: 2) were intravenously injected from the tail vein at the same schedule and dose as those in the miR-4711-5p administration group. In the untreated group, no medicine was administered. The tumor size (long diameter × short diameter × short diameter × 1/2) of the back of the mouse and the body weight of the mouse were measured over time during the test period. On day 14, the tumor was removed from the mouse, and the size was observed. Furthermore, on day 14, the brain, the heart, the lung, the liver, the kidney, the spleen, and the colon were excised from the mice, hematoxylin/eosin staining was performed, and tissue observation was performed.

The result of measuring the tumor size over time is shown in Fig. 19, and the result of observing the tumor excised from the mouse on Day 14 is shown in Fig. 20. As a result, in the miR-4711-5p administration group, the tumor size was significantly smaller than that in the negative control group and the untreated group.

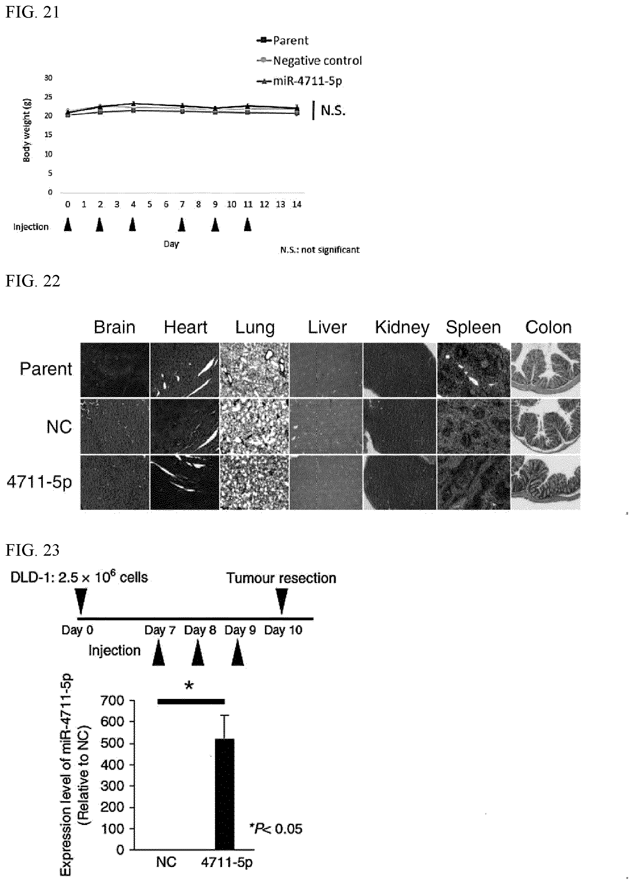

In addition, a result of measuring the body weights of mice over time are shown in Fig. 21. As a result, it was confirmed that there was no significant difference in body weight among the three groups and there was no side effect by miR-4711-5p.

In addition, on day 14, the brain, the heart, the lung, the liver, the kidney, the spleen, and the colon were removed from the mouse, and the result of hematoxylin-eosin staining is shown in Fig. 22. From the results of hematoxylin and eosin staining of each tissue, no side effect by miR-4711-5p was observed.

### Test Example 19: Evaluation of antitumor effect of miR-471 1-5p

On the left and right sides of the back of eight-week-old BALB/cA nude mice (Female, manufactured by CLEA Japan, Ltd.), DLD-1 was subcutaneously injected at 2.5 × 10⁶ cells/tumor to prepare model mice having a solid tumor. Mice were randomly divided into two groups of three mice in a miR-4711-5p administration group (miR-4711-5p) and three mice in a negative control group (Negative control). In the miR-4711-4711-5p administration group, the time point at which DLD-1 was subcutaneously injected was defined as day 0, and on days 7, 8 and 9, carbonate apatite nanoparticles containing iR-4711-5p (prepared under the same conditions as in Test Example 18) were intravenously injected from the tail vein so as to be 40 µg/dose in terms of miR-5p amount. In the negative control group, carbonate apatite nanoparticles containing a control miRNA (SEQ ID NO: 2) were intravenously injected from the tail vein at the same schedule and dose as those in the miR-4711-5p administration group. Tumors were removed from the mice on day 10.

The amount of miR-4711-5p in the excised tumor was measured by PCR. In addition, mRNA amounts of KLF5, TFDP1, and MDM2 in the extracted tumor were measured by PCR. Furthermore, all proteins were extracted from the extracted tumor, and the expression levels of KLF5, TFDP1, MDM2, cPARP (cleaved PARP), cCaspase3 (cleaved Caspase3), and ACTB (β-actin) at the protein level were measured by Western blotting. Furthermore, for the excised tumors, fragmented DNA generated by apoptosis was stained by TUNEL assay, and nuclei were stained by Hoechst.

The results of measuring miR-4711-5p levels in tumors are shown in Fig. 23. As a result, in the miR-4711-5p administration group, the amount of miR-4711-5p in the tumor was significantly increased as compared with the negative control group.

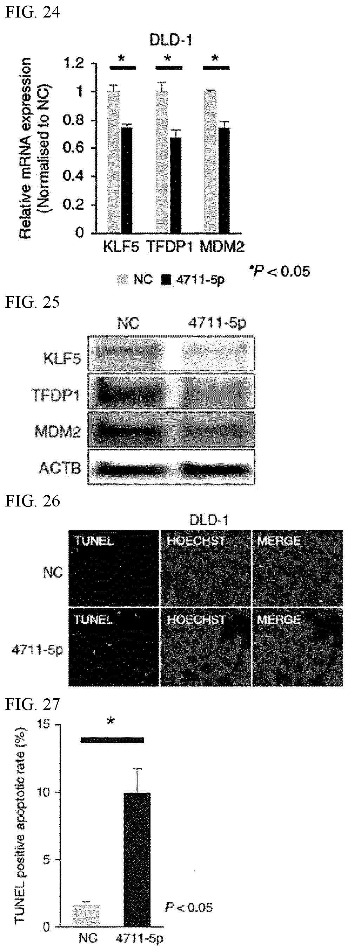

In addition, the results of measuring the mRNA amounts of KLF5, TFDP1, MDM2, and ACTB in the tumor are shown in Fig. 24, and the results of measuring the protein amounts of KLF5, TFDP1, MDM2, and ACTB in the tumor are shown in Fig. 25. As a result, it was confirmed that in the miR-4711-5p administration group, the expression levels of KLF5, TFDP1, and MDM2 were reduced in both mRNA and protein as compared with the negative control group.

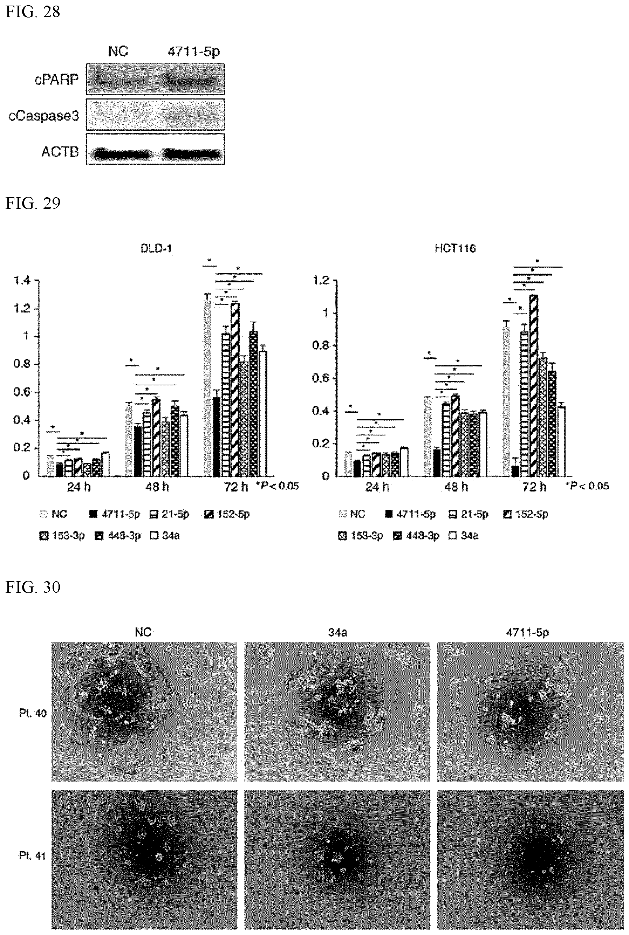

The results of the TUNEL assay are shown in Figs. 26 and 27, and the results of measuring the expression of cPARP, cCaspase3, and ACTB at the protein level are shown in Fig. 28. As a result, it was found that in the miR-4711-5p administration group, the number of cells induced to apoptosis was significantly increased, and the expression levels of the apoptotic proteins cPARP and cCaspase3 were increased.

### Test Example 20: Cell growth test (comparison with other miRNAs)

Using colorectal cancer cell lines (DLD-1 and HCT116), a cell growth test was performed on four kinds of miRNAs (miR-21-5p, miR-152-5p, miR-153-3p, and miR-448-3p) of which the expression suppressing action of miR-4711-5p and KLF5 has been reported, and miR-34a of which the antitumor effect has been reported. Specifically, first, two colorectal cancer cell lines of DLD-1 and HCT116 were seeded in wells of a 96-well plate at 3.0 × 10³ cells/well, followed by overnight culture, and then miR-4711-5p, miR-21-5p, miR-152-5p, miR-153-3p, miR-448-3p, miR-34a, and a negative control miRNA (NC, SEQ ID NO: 2) were transfected using Lipofectamine 2000 (Invitrogen). After 24, 48, and 72 hours from the transfection, the cell survival number was measured with GloMax-Multi+ Detection System (Promega) using cell counting kit-8 (DOJINDO LABORATORIES).

The results are shown in Fig. 29. Note that in Fig. 29, the vertical axis represents the absorbance at 450 nm. As a result, the highest growth inhibitory effect was observed when the transfection of miR-4711-5p was performed.

### Test Example 21: Cell growth test (verification using colorectal cancer cells collected from patient)

A cell growth test was performed using colorectal cancer cells collected from five patients (Pt. 28, Pt. 36, Pt. 40, Pt. 41, and Pt. 45) in stages II and III. This test was performed using colorectal cancer cells collected from five colorectal cancer patients who obtained informed consent under the permission of the Ethics Committee of Osaka University Hospital. Spheroids of colorectal cancer cells collected from each patient were dissociated into single colorectal cancer cells by incubation with trypLE express (Invitrogen) at 37°C for 30 minutes. The obtained single colorectal cancer cells were seeded in wells of a 96-well plate at 4.0 × 10³ cells/well, followed by overnight culture, and then transfected with miR-4711-5p, miR-34a, and a negative control miRNA (NC, SEQ ID NO: 2) using Lipofectamine 2000 (Invitrogen). After 72 hours from the transfection, the state of cells was observed, and the cell survival number was measured using cell counting kit-8 (DOJINDO LABORATORIES) using GloMax-Multi+ Detection System (Promega).

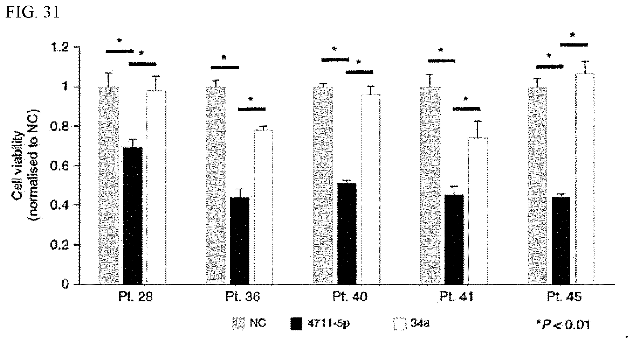

The results of observing the state of cells are shown in Fig. 30, and the results of measuring the number of cell surviving cells are shown in Fig. 31. As a result, it was confirmed that the growth inhibitory effect of miR-4711-5p on colorectal cancer cells obtained from the colorectal cancer patients was significantly higher than that of miR-34a and a negative control (p < 0.01 p).

### <Comprehensive discussion>

From the results of Test Examples 1 to 9, the following points were revealed, and it was confirmed that miR-4711-5p has an antitumor effect.
(1) Among miRNAs against KLF5, miR-4711-5p can effectively reduce the viability of cancer cells and suppress the expression of KLF5 in cancer cells.
(2) miR-4711-5p binds to the 3'UTR of KLF 5.
(3) miR-4711-5p can suppress the growth, invasion, and migration of cancer cells.
(4) miR-4711-5p can induce the apoptosis of cancer cells.

In addition, from the results of Test Example 10 to 12 described above, the following points were revealed, and it was confirmed that miR-4711-5p reduces the sternness of cancer cells.
(5) miR-4711-5p can suppress the expression of stem cell markers of cancer stem cells.
(6) miR-4711-5p increases the percentage of cancer cells with high ROS activity.
(7) miR-4711-5p can suppress the formation of spheres in cancer cells.

In addition, from the results of Test Example 13 to 17 described above, the following points were revealed, and it was confirmed that miR-4711-5p can suppress the transition from G1 phase to S phase in the cell cycle of cancer cells.
(8) miR-4711-5p increases the percentage of G0/G1 phase cancer cells.
(9) One of the mechanisms by which the transition from G1 phase to S phase is suppressed by miR-4711-5p is downregulation of TFDP1 directly targeted by miR-4711-5p.
(10) One of the mechanisms by which the transition from G1 phase to S phase is suppressed by miR-4711-5p is downregulation of MDM2 directly targeted by miR-4711-5p.

From the results of Test Examples 18 and 19, it was confirmed that the miR-4711-5p exhibited an antitumor effect also in vivo, and no side effect on normal tissues was observed.

From the results of Test Examples 20 and 21, it was confirmed that the miR-4711-5p had an antitumor effect superior to that of the miR-34a reported to have an antitumor effect, and actually exhibited an excellent antitumor effect on colorectal cancer cells extracted from the colorectal cancer patients.

## Claims

1. An agent for use in a method of treating cancer comprising miR-4711-5p as an active ingredient.

2. The agent according to claim 1, wherein the miR-4711-5p is a mature miRNA, pri-miRNA, or pre-miRNA.

3. The agent according to claim 1 or 2, wherein the miR-4711-5p is complexed to a carbonate apatite particle.

4. An agent for use in a method of suppressing growth of cancer stem cells comprising miR-4711-5p as an active ingredient.

5. Use of miR-4711-5p for the manufacture of an agent for use in a method of treating cancer.

6. miR-4711-5p for use in a method of treating cancer.

7. A method of treating cancer, comprising administering a therapeutically effective amount of miR-4711-5p to a cancer patient.

8. Use of miR-4711-5p for the manufacture of an agent for use in a method of suppressing growth of cancer stem cells.

9. miR-4711-5p for use in a method of suppressing growth of cancer stem cells.

10. A method of suppressing growth of cancer stem cells, comprising administering a therapeutically effective amount of miR-4711-5p to a cancer patient to suppress growth of cancer stem cells.


SEQUENCE LISTING

<110>  CancerStem Tech Inc.
       Osaka University

<120>  Agent for treating cancer

<130>  20014WO

<150>  JP2019-105500
<151>  2019-6-5

<160>  7

<170>  PatentIn version 3.5

<210>  1
<211>  23
<212>  RNA
<213>  Homo sapiens

<400>  1
ugcaucaggc cagaagacau gag                                               23


<210>  2
<211>  19
<212>  RNA
<213>  Artificial Sequence

<220>
<223>  Negative control miRNA

<400>  2
auccgcgcga uaguacgua                                                    19


<210>  3
<211>  23
<212>  RNA
<213>  Homo sapiens

<400>  3
aauaguaaug ugaugcugau gcu                                               23


<210>  4
<211>  22
<212>  RNA
<213>  Homo sapiens

<400>  4
gacaauguug cauuuaugau gc                                                22


<210>  5
<211>  23
<212>  RNA
<213>  Homo sapiens

<400>  5
caaaacguug aauugaugau gca                                               23

<210>  6
<211>  23
<212>  RNA
<213>  Homo sapiens

<400>  6
uuugauacca gugugcugau gca                                                23

<210>  7
<211>  23
<212>  RNA
<213>  Homo sapiens

<400>  7
uauggaauaa aacuacugau gca                                                23